# EUROPEAN PATENT SPECIFICATION

(11) **EP 1 891 939 B1**
(45) Date of publication and mention of the grant of the patent: **11.09.2013**
(21) Application number: 06291236.5
(22) Date of filing: 28.07.2006
(51) Int. Cl.: A61K 9/00

(54) **Compositions containing quaternary ammonium compounds**
Zusamensetzungen die quaternäre Ammonium Verbindungen enthalten
Compositions comprenant des composés ammonium quaternaires

(43) Date of publication of application: 27.02.2008
(73) Proprietor: Novagali Pharma S.A., 91000 Evry (FR)
(72) Inventor: Rabinovich-Guilatt, Laura, 75015 Paris (FR); Lambert, Grégory, 92290 Châtenay Malabry (FR); Lallemand, Frédéric, 94260 Fresnes (FR); Philips, Betty, 92160 Antony (FR)
(74) Representative: Icosa

(56) References cited:
- EP-A2- 0 642 782
- WO-A-95/31958
- WO-A-97/25975
- GB-A- 633 175
- US-A- 3 954 644
- US-A- 5 951 988

## Description

This invention relates to pharmaceutical, ophthalmic or cosmetic compositions containing quaternary ammonium compounds, more preferably to ophthalmic emulsions being useful for eye care or for the treatment of eye conditions. This invention also relates to compositions including at least one quaternary ammonium compound as cationic agent.

Quaternary ammonium compounds are organic compounds usually used as an antiseptic or antimicrobial agent. For example, benzalkonium chloride is a nitrogenous cationic surface-acting agent belonging to the quaternary ammonium group. Benzalkonium chloride is generally defined as a mixtures of compounds of general formula C₆H₅CH₂N (CH₃) ₂RCl, wherein R is a C12-C24 alkyl group.

Benzalkonium chloride, as usually provided by the manufacturers wanting to comply with the European and/or American Pharmacopeia, is a mixture of n-alkyl dimethyl benzyl ammonium chlorides of various alkyl chain lengths. For example, FeF Chemicals A/S (Denmark) supplies, under reference 8100301U (BAK USP/NF), a mixture of three alkyl dimethyl benzyl ammonium chlorides including : (1) 60-70% of C₁₂-alkyl dimethyl benzyl ammonium chloride (2) 30-40% of C₁₄-alkyl dimethyl benzyl ammonium chloride, and less than 5% of C₁₆-alkyl dimethyl benzyl ammonium chloride

Benzalkonium chloride, as a mixture of alkyl dimethyl benzyl ammonium having various alkyl chain lengths is used as preservative agent in topical ophthalmic products. Benzalkonium chloride also has cationic agent properties, and was used as cationic agents for emulsions, especially ophthalmic emulsions.

When mixtures of benzalkonium chlorides having various alkyl chain lengths are used in emulsions, they may act both as preservative agents and cationic agents.

The Applicant worked on long chain quaternary ammonium compounds, and noticed that the length of the alkyl chain was important with regards to the function performed by the quaternary ammonium compounds: acting on the length of the alkyl chain resulted in enhancing or reducing the cationic power of the quaternary ammonium compounds. Without wanting to be linked by any theory, the Applicant observed on working on oil-in-water emulsions, that long chain quaternary ammonium compounds are preferentially localized at the oil/water interface of the emulsions, resulting in (1) emulsions with higher zeta potential and (2) more stable emulsions. As quaternary ammonium may be considered as undesirable or toxic, it is thus a goal of this invention to provide cationic composition having a reduced content of quaternary ammonium compound.

The Applicant also observed that, in emulsions, quaternary ammonium compounds having long alkyl chains, for example quaternary ammonium compounds having C14-C18 alkyl chains, when compared to C12-alkyl chains, did not have a good bactericidal activity, whereas they conferred a greatest cationic power.

Moreover, the Applicant observed that long chain quaternary ammonium compounds were present preferentially at the oil/water interface of the emulsion droplets, and less in the aqueous phase. The fact that quaternary ammonium compounds may be present in the aqueous phase in a very small amount only, or not present, leads to a loss of preservative effect or poor preservative effet, as well as to less toxic emulsions.

Thus, one of the goals of this invention is to provide stable cationic emulsions comprising a reduced amount of benzalkonium chlorides, and still using said benzalkonium chlorides as a source, or the only source, of cationic agents, said emulsions being preserved or not.

Preferably, the emulsions of the invention are useful for cosmetic or ophthalmic purposes.

Another goal of this invention is to provide ammonium halide compositions, preferably benzalkonium compositions, suitable for the preparation of cationic emulsions. Preferably, said cationic emulsions are useful for ophthalmic or cosmetic purposes.

This invention thus relates to a composition comprising at least one quaternary ammonium halide, more preferably ammonium chloride or bromide, in which the nitrogen atom of the ammonium group is substituted by at least one alkyl group having at least 12 carbon atoms, said composition including:
a) at least 20% in weight by weight of the total composition, of ammonium halides in which the nitrogen atom is substituted by at least one alkyl group having at least 14 carbon atoms, preferably 14 or 16 carbon atoms and
b) more than 5 %, preferably more than 7 % in weight by weight of the total composition, of ammonium halides in which the nitrogen atom is substituted by at least one alkyl group having at least 16 carbon atoms.

According to an embodiment of the invention, the composition includes at least 20% w/w of the total composition, of C14-alkyl ammonium halides and at least 10%, preferably at least 15%, more preferably at least 20 % w/w of the total composition, of C16-alkyl ammonium halides.

According to another embodiment, the composition includes as only alkyl ammonium halide, C16- alkyl ammonium halide, preferably C16-alkylbenzyldimethyl ammonium halide.

Preferably, the ammonium halides used in this invention are benzyl dimethyl ammonium chlorides or bromides, wherein the nitrogen atom is further substituted by an alkyl group having at least 12 carbon atoms, preferably 12, 14 and/or 16 carbon atoms.

According to an embodiment of the invention, the composition comprises C14- and C16-alkyl benzyl dimethyl ammonium chlorides. In a further embodiment, the composition of the invention does not include any C12-alkyl ammonium halide.

According to another embodiment, the composition of the invention includes trimethyl ammonium chloride or bromide, wherein the nitrogen atom is further substituted by an alkyl group having at least 12 carbon atoms, preferably 12 and/or 14 and/or 16 carbon atoms. According to an embodiment, the trimethyl ammonium chloride or bromide is cetyltrimethylammonium bromide.

According to a further embodiment, and whatever the ammonium halides are, the amount of ammonium halides in which the nitrogen atom is substituted by at least one alkyl group having 14 or 16 carbon atoms may preferably represent at least 50% w/w of the total amount of all ammonium halides present in the composition, this percentage being in dry weight.

According to another embodiment the amount of ammonium halides in which the nitrogen atom is substituted by at least one alkyl group having at least 16 carbon atoms may preferably represent more than 90% w/w measured in dry weight of the total amount of all ammonium halides present in the composition.

According to another embodiment the amount of ammonium halides in which the nitrogen atom is substituted by at least one alkyl group having at least 16 carbon atoms may preferably represent more than 30% w/w measured in dry weight of the total amount of all ammonium halides present in the composition.

According to an embodiment of the invention, the composition includes, dry weight by total dry weight, of all ammonium halides present in the composition, 40% of ammonium halides in which the nitrogen atom is substituted by an alkyl group having 12 carbon atoms, 30 % of ammonium halides in which the nitrogen atom is substituted by an alkyl group having 14 carbon atoms and 30 % of ammonium halides in which the nitrogen atom is substituted by an alkyl group having 16 carbon atoms; preferably, the composition includes, measured in dry weight, in weight by total weight of all ammonium halides present in the composition, a mixture of 40 % w/w of BAK C12, 30 % w/w of BAK C14, and 30 % w/w of BAK C16.

In another embodiment, the composition includes, in dry weight by total dry weight, a mixture of 40 % w/w ATAB C12, 30 % w/w ATAB C14 or 30 % w/w ATAB C16.

In another embodiment, the mean molecular weight of the ammonium halides present in the composition is less than 372, this calculation based on the total alkyl basis.

In another embodiment, the composition consists of, in dry weight by total dry weight of the halide ammonium composition, 85% of ammonium halides in which the nitrogen atom is substituted by an alkyl group having 14 carbon atoms and 15% of ammonium halides in which the nitrogen atom is substituted by an alkyl group having 16 carbon atoms.

In another embodiment, the composition consists of 45% of ammonium halides in which the nitrogen atom is substituted by an alkyl group having 12 carbon atoms and 55% ammonium halides in which the nitrogen atom is substituted by an alkyl group having 16 carbon atoms

According to an embodiment, the weight ratio of C12-alkyl ammonium halides to the sum of C14-alkyl ammonium halides and C16-alkyl ammonium halide, is less than 1.5, preferably less than 1.35, more preferably less than 1.20. More preferably, the weight ratio of BAK C12 to the sum of BAK C14 and BAK C16, is less than 1.5, preferably less than 1.35, more preferably less than 1.20.

According to an embodiment of the invention, the composition includes more than one ammonium halide: in a first embodiment the composition includes three ammonium halides, preferably a C12-alkyl ammonium halide and a C14-alkyl ammonium halide and a C16-alkyl ammonium halide, more preferably BAK C12 and BAK C14 and BAK C16 ; in another embodiment, the composition includes two ammonium halides, preferably a C14-alkyl ammonium halide and a C16-alkyl ammonium halide, preferably BAK C12 and BAK C16 ; in another embodiment of the invention, the composition includes only one ammonium halide, preferably C16-alkyl ammonium halide, more preferably BAK C16.

This composition is obtained by mixing various components obtained from commercial source, or by de novo synthesis of the composition itself, or by purification of commercial products.

In the meaning of this invention,
"Cationic emulsions" are emulsions having a positive zeta potential, preferably a zeta potential higher to 10 mV;
"long alkyl chain" are alkyl moieties having at least 14 carbon atoms;
"quaternary ammonium compounds" refer to ammonium halides
in which the nitrogen atom is substituted by at least one alkyl group having at least 12 carbon atoms ; quaternary ammonium compounds also, but not exclusively, include n-alkyl dimethyl benzyl ammonium chloride also called benzalkonium chloride (hereafter also referred to as BAK or ADBAC); n-alkyl dimethyl benzyl ammonium bromide; n-alkyl trimethyl ammonium bromide (also referred to as ATAB), n-alkyl meaning an alkyl group of at least 12 carbon atoms;
"C14-alkyl ammonium halides" means ammonium halides in which the nitrogen atom of the ammonium group is substituted by at least one alkyl group having at least 14 carbon atoms.
"BAK C12" refers to benzododecinium chloride (CAS 139-07-1); "BAK C14" refers to myristalkonium chloride (CAS 139-08-2); "BAK C16" refers to cetalkonium chloride (CAS 122-18-9) ;
"ATAB C12" refers to lauryl trimethyl ammonium bromide (CAS 1119-94-4); "ATAB C14" refers to Myristil trimethyl ammonium bromide (CAS 1119-97-7); "ATAB C16" or "CTAB" refers to Cetyl trimethyl ammonium bromide (CAS 57-09-0),
"MCT" means Medium chain triglycerides ; for the experimentation, TCM^{™} (Societé des Oleagineux, France) was the MCT used;
"ND" means "not determined".

The invention also relates to a cationic oil-in-water emulsion comprising a composition of the invention, as described hereabove. According to an embodiment of the invention, the oil-in-water emulsion comprises a composition as described above, said emulsion comprising 0.0005 to 0.1% of quaternary ammonium halides. By cationic oil-in water emulsion is understood an oil-in-water emulsion having a positive zeta potential. The emulsion of the invention has a positive zeta potential and is stable, which means that it keeps a positive zeta potential overtime. In a preferred embodiment, the oil-in-water emulsion according to the invention includes droplets of size 100 to 500 nm, preferably 110 to 250 nm.

In a first embodiment, the oil-in-water emulsion of the invention is for cosmetic use. Preferably, the emulsion of the invention is intended for making up or caring for the body and face skin, including the lips, or for hair care. The cosmetic emulsion of the invention can be a product for caring for the skin, such as a care base for the skin, a care cream (e.g., day cream, night cream, anti-wrinkle cream), a make-up base or a composition for caring for the lips (e.g., lip balm), or make-up remover, including eye make-up remover. The product of the invention may also be used for enhancing moisture of hair and/or skin.

In a preferred embodiment, the oil-in-water emulsion of the invention is useful for eye care or for the treatment of eye diseases or eye conditions.

In the meaning of the invention, eye diseases or eye conditions means a wide variety of ocular conditions such as glaucoma, ocular inflammatory conditions such as keratitis, uveitis, intra-ocular inflammation, allergy and dry-eye syndrome ocular infections, ocular allergies, cancerous growth, neo vessel growth originating from the cornea, retinal oedema, macular oedema, diabetic retinopathy, retinopathy of prematurity, degenerative diseases of the retina (macular degeneration, retinal dystrophies), retinal diseases associated with glial proliferation.

More preferably, the oil-in-water emulsion according to the invention comprises:
a) an oil phase,
b) 0.0005 to 0.1% w/w of a composition of quaternary ammonium halides, which contain at least 20% w/w, of ammonium halides in which the nitrogen atom is substituted by at least one alkyl group having at least 14 carbon atoms, preferably 14 or 16 carbon atoms and more than 5 % w/w, preferably more than 7 % w/w of ammonium halides in which the nitrogen atom is substituted by at least one alkyl group having at least 16 carbon atoms, in weight by weight of the total emulsion
c) surfactants,
d) optionnaly antioxidants, isotonicity, viscosifying, pH adjusting, buffering, preservative, solubilizers, chelating, thickener agents,
e) water.

According to an embodiment of the invention, the oil-in-water emulsion further comprises hydroxypropyl guar or polyéthylene glycol-400 or a mixture of both.

According to an embodiment of the invention, the emulsion includes an oil phase, surfactants such as for example tyloxapol or poloxamer or tocopherol polyethyleneglycol succinate or polysorbate 80 or any suitable surfactant, and 0.0005% to 0.1 %w/w preferably 0.001 to 0.005 % w/w of the total composition, of a composition of ammonium halides according to the invention, as described hereabove.

According to an embodiment of the invention, the emulsion includes C12-alkyl benzyl dimethyl ammonium chloride or bromide, C14-alkyl benzyl dimethyl ammonium chloride or bromide, and C16-alkyl benzyl dimethyl ammonium chloride or bromide. According to another embodiment of the invention, the emulsion comprises C14- and C16-alkyl benzyl dimethyl ammonium chlorides. In a further embodiment, the emulsion of the invention does not include any C12-alkyl ammonium chloride or bromide. According to an embodiment, the oil-in-water emulsion comprises C16-alkyl quaternary ammonium halide as only source of quaternary ammonium halide.

According to another embodiment, the emulsion of the invention includes trimethyl ammonium chlorides or bromides, wherein the nitrogen atom is further substituted by an alkyl group having at least 12 carbon atoms; or by an alkyl group having at least 14 carbon atoms; or by an alkyl group having at least 16 carbon atoms; or by a mixture of such trimethyl ammonium chlorides or bromides.

According to a preferred embodiment, the emulsion of the invention includes MCT, glycerol, tyloxapol and poloxamer, and a composition of the invention including at least one ammonium halide as hereabove described.

Preferably, the emulsion includes 1 to 2 % of oil phase, preferably of MCT, castor oil or mineral oil.

Preferably, the emulsion includes 0.1 to 1 % of surfactants, preferably tyloxapol and/or poloxamer and/or polysorbate 80 and/or tocopherol polyethyleneglycol succinate.

In a preferred embodiment, the emulsion includes an oil phase, preferably 2% MCT or 1% mineral oil, and surfactants, preferably 0.3% Tyloxapol and 0.1% Poloxamer, optionally antioxidants such as alpha-tocopherol and optionally isotonicity agents such as mannitol or glycerol, and a composition of ammonium halides, preferably BAK C12, BAK C14, BAK C16 or a mixture of at least two thereof, or in another embodiment ATAB C12, ATAB C14 or ATAB C16 or a mixture of at least two thereof, said ammonium halides composition being in a concentration ranging from 0.0005 to 0.1% w/w of the total emulsion.

According to a first embodiment, the emulsion does not contain any active principle. In this embodiment, the emulsion is particularly useful as artificial tears, or for the treatment of dry eye condition such as for example Dry Eye Syndrome or Chronic Dry Eye Disease (CDED), both clinically known as keratoconjuctivitis sicca.

According to a second embodiment, the composition of the invention contains an active principle, preferably chosen among antibiotics such as aminoglycosides, carbacephem, carbapenems, cephalosporins, glycopeptides, penicillins, polypeptides, quinolones, sulfonamides, tetracyclines and others; antiviral agents such as cidofovir, ganciclovir, valaciclovir or acyclovir; antifungals such as polyene antibiotics, imidazole and triazole, allylamines, ; intraocular pressure lowering agents such as alpha-adrenergic agonists, beta-adrenergic blockers, carbonic anhydrase inhibitors, cannabinoids, prostaglandins, prostaglandins analogues, derivatives and prodrugs; anti-inflammatory agents such as COX-2 inhibitors, salicylates, 2-arylpropionic acids, N-arylanthranilic acids, oxicams, sulphonanilides, pyrazolidines derivatives, arylalkanoic acids, 3-benzolphenylacetic acids and derivatives; steroids such as cortisone, hydrocortisone, prednisone, prednisolone, methylprednisone, fluoromethalone, medrysone, betamethasone, loteprednol, flumethasone, mometasone, testosterone, methyltestosterone, danazol, beclomethasone, dexamethasone, dexamethasone palmitate, tramcinolone, triamcinolone acetonide, fluocinolone, fluocinolone acetonide and difluprednate ; antiallergic compounds such as olapatadine, ketotifen, azelastine, epinastine, emedastine, levocabastive, terfenadine, astemizole and loratadine; anti-angiogenic compounds such as thalidomide, VEGF inhibitors, VEGF soluble receptors, VEGF-traps, VEGF-antibodies, anti VEGF-siRNA; biological agents such as antibodies or antibodies fragments, oligoaptamers, aptamers and gene fragments, oligonucleotides, plasmids, ribozymes, small interfering RNA, nucleic acid fragments, peptides and antisense sequences; growth factors such as epidermal growth factor, fibroblast growth factor, platelet derived growth factor, transforming growth factor beta, ciliary neurotrophic growth factor, glial derived neurotrophic factor, NGF, EPO and P1GF; immunomodulating agents such as glucocorticoids, drugs acting on immunophilins, interferons, opioids ; cytostatics such as alkylating agents, antimetabolites and cytotoxic antibiotics; antioxidants such as alpha-tocopherol, ascorbic acid, retinoic acid, lutein and their derivatives, precursors or prodrugs ; UV-filter compounds such as benzophenones; anti-redness agents such as naphazoline, tetrahydrozoline, ephedrine and phenylephrine; fatty acids such as omega-3 fatty acids.

Preferably, the composition of the invention contains an active principle chosen among the group consisting of ganciclovir, acyclovir, ketoconazole, amphotericin B, brimonidine, dexanabinol, forskolin, travoprost, latanoprost, amfenac, diclofenac, flurbiprofen, flurbiprofen axetyl, ketorolac, dexamethasone palmitate, rimexolone, triamcinolone, difluprednate, fluocinolone, olapatadine, alpha-tocopherol, vitamin A, vitamin C, lutein, eicosapentaenoic acid, docosahexaenoic acid, octylmethoxycinnamate, benzophenone-3, octyl dimethyl PABA, cyclosporine A, mycophenolate, sirolimus and tacrolimus and/or their derivatives; and/or their prodrugs; and/or their precursors; and/or acceptable salts thereof; alone or in combination.

In an embodiment of the invention, the oil-in-water emulsion is preserved.

In another embodiment of the invention, the oil-in-water emulsion is unpreserved; in an embodiment, the emulsion is packaged in unitary doses; in another embodiment, the emulsion is packaged in suitable multidose containers.

The invention relates to a medicament comprising an oil-in-water emulsion as described above or a composition as described above.

The invention also relates to the use of an oil-in-water as described above or a composition as described above for the manufacture of a medicament or an ophthalmic composition the treatment of an eye condition or disease.

The invention also relates to a cosmetic composition comprising an oil-in-water emulsion as described above or a composition as described above.

The invention also relates to a non-therapeutical process for caring for, removing makeup from and/or cleansing the skin, the lips and/or the eyes, and/or for hair care, comprising applying an oil-in-water emulsion as described above or a composition as described above to the skin, the lips, the eyes, and/or the hair.

The following examples and figures illustrate the invention and should not be interpreted in any way as reducing the scope of this invention.

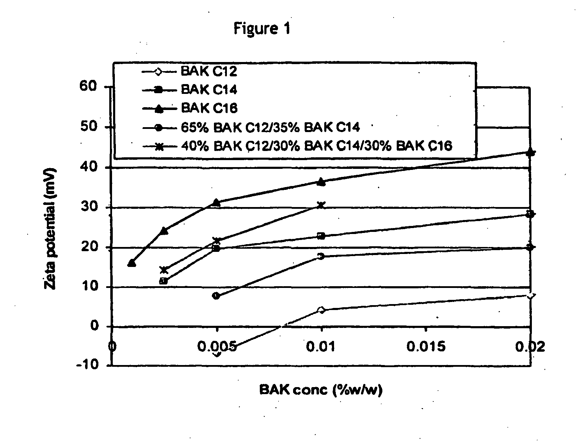

Fig.1 refers is a graph showing zeta potential values (mV) of the emulsion of the invention depending of various BAK concentrations, and is to be read in connexion with table 2 of the examples.
Fig. 2 refers to a graph showing the unexpected decrease of the toxicity of the emulsion comprising BAK C16.

### Examples:

All concentrations in the emulsion formulae are expressed in weight/weight of the entire formulation percentages, unless stated differently.

### 1. Emulsions composition

Emulsions containing different amounts and chain lengths of BAK and ATAB were prepared. They contained 2% MCT or 1% mineral oil as oil phase, 0.3% Tyloxapol and 0.1% Poloxamer as surfactants. They could also contain antioxidants such as alpha-tocopherol and isotonicity agents such as mannitol or glycerol. Concentrations ranging from 0.001 to 0.1% of BAK C12, BAK C14, BAK C16 or a mixture of all, and from 0.0025 to 0.005% of ATAB C12, ATAB C14 or ATAB C16 were prepared.

### 2. Emulsions preparation

The oily and the water phases of the emulsion, which might contain or not an active principle, may be separately heated to an appropriate temperature. This temperature may be the same in both cases. Surfactants might be dissolved in the oil, water phase or in both. A first coarse emulsion is generated by magnetic stirring, and the droplet size is reduced by high shear mixing, high pressure homogenization, or both.

The oil-in-water emulsions of the present invention can be sterilized after preparation using heat, for example, autoclave steam sterilization.

### 3. Impact of chain length on emulsions characteristics

### a) Emulsion droplet size

The mean diameter of the oil droplets is determined by dynamic light scattering using a High Performance Particle Sizer type HPPS 5001 (Malvern Instruments, Worcestershire, UK). Measurements are performed at 25°C following dilution of the emulsion in double distilled water.

**Table 1: Emulsions droplet size values (nm)**

| | 0.001% | 0.0025% | 0.005% | 0.01% | 0.02% | 0.04% | 0.1% |
|---|---|---|---|---|---|---|---|
| ATAB C12 | - | - | - | | - | | - |
| ATAB C14 | - | 203 | - | - | - | - | - |
| ATAB C16 | - | 222 | 212 | - | - | - | - |
| BAK C12 | - | - | 198 | 263 | 230 | 225 | 180 |
| BAK C14 | - | 204 | 190 | 190 | 155 | 238 | 185 |
| BAK C16 | 220 | 210 | 148 | 180 | 155 | 188 | 183 |
| 65% BAK C12 | | | | | | | |
| 35% BAK C14 | - | - | 357 | 397 | 190 | 180 | 156 |
| 40% BAK C12 | | | | | | | |
| 30% BAK C14 | | | | | | | |
| 30% BAK C16 | - | 220 | 210 | 145 | - | - | - |

### b) Emulsion zeta potential

Zeta potential can be measured by a zetameter such as Zetasizer 2000, Malvern Instruments Ltd, UK. The zeta potential of the emulsion droplet surface is determined by electrophoretic mobility. Measurements are performed at 25°C following dilution at 1:250 of the emulsion in double distilled water. The electrophoretic mobility is converted into zeta potential values through the Smoluchowsky equation.

The following table and graph show the evolution of the zeta potential (indicative of the surface change) at increasing concentrations of quaternary ammonium. It can be observed that for more lipophilic (longer) chain lengths, positive charges are attained more rapidly and at slower concentrations, suggesting a preferential partition within the oil droplet surface.

**Table 2: Emulsions zeta potential values (mV)**

| | 0.001% | 0.0025% | 0.005% | 0.01% | 0.02% | 0.04% | 0.1% |
|---|---|---|---|---|---|---|---|
| ATAB C12 | - | -35.5 | -14.6 | - | - | - | - |
| ATAB C14 | - | -11.4 | -8.0 | - | - | - | - |
| ATAB C16 | - | +11.9 | +20.2 | - | - | - | - |
| BAK C12 | - | - | -6.9 | +4.2 | +7.9 | +16.8 | +23.8 |
| BAK C14 | - | +11.4 | +19.6 | +22.9 | +28.4 | +393 | +44.5 |
| BAK C16 | +16.2 | +24.4 | +31.4 | +36.7 | +44.1 | +47.2 | +48.9 |
| 65% BAK C12 | | | | | | | |
| 35% BAK C14 | - | - | +7.6 | +17.7 | +20.0 | +35.0 | +40.3 |
| 40% BAK C12 | | | | | | | |
| 30% BAK C14 | | | | | | | |
| 30% BAK C16 | - | +14.3 | +21.6 | +30.7 | - | - | - |

### 3. Emulsion stability over time

The stability of the emulsions can be evaluated by the evolution of their aspect, with a visual score with a visual score going from 13 - best aspect to 1- total phase separation.

It can be observed from the following table that, at equimolar concentration, longer (more lipophilic) chain length QA results in more stable emulsion.

| Emulsions | Type and conc. of QA | After preparation (T0) | Following 3 months at 40°C |
|---|---|---|---|
| Z01EM207 | 0.25 mM BAK C12 | 12 | 2 |
| Z01EM208 | 0.25 mM BAK C14 | 13 | 7 |
| Z01EM209 | 0.25 mM BAK C16 | 13 | 9 |
| Z01EM204 | 0.5 mM BAK C12 | 10 | 2 |
| Z01EM205 | 0.5 mM BAK C14 | 13 | 7 |
| Z01EM206 | 0.5 mM BAK C16 | 11 | 9 |

### 4. Impact of chain length on antimicrobial activity of QA

The antimicrobial effectiveness of the emulsions and solutions of BAK C12, BAK C14 and BAK C16 at equimolar concentrations corresponding to 0.005% w/w BAK C12 has been determined according to the chapter 51 of the United States Pharmacopeia.

**Table 3: Antimicrobial effectiveness testing of emulsions and solutions containing BAK C12. C14 or C16.**

| Chain length | BAK C₁₂ | BAK C₁₄ | BAK C₁₆ |
|---|---|---|---|
| Solution | ✔ (SOL226) | NA | ✔ (SOL254) |
| Emulsion | ✔ (EM212) | ✔ (EM219) | × (EM234) |

| | | | |
|---|---|---|---|
| ✔ : preserved, × : not preserved, NA : not assessed | | | |

### 5. Impact of chain length on toxicity of QA

The ocular irritation of the emulsions and solutions has been evaluated using an adaptation of the Draize test on white male New Zealand rabbits (2.75-3.00 kg). Fifty µL of emulsion or solution were instilled unilaterally and 50 µL of NaCl 0.9% in the other eye of the three rabbits per group. General aspect assessment of the animals was performed (behaviour, blinking, itching of the eye with forelegs) as well as eye tissue evaluation (conjunctiva, cornea, iris) after instillation, 1, 24, 48 and 72 hours. Observations were scored according to the Draize test protocol.

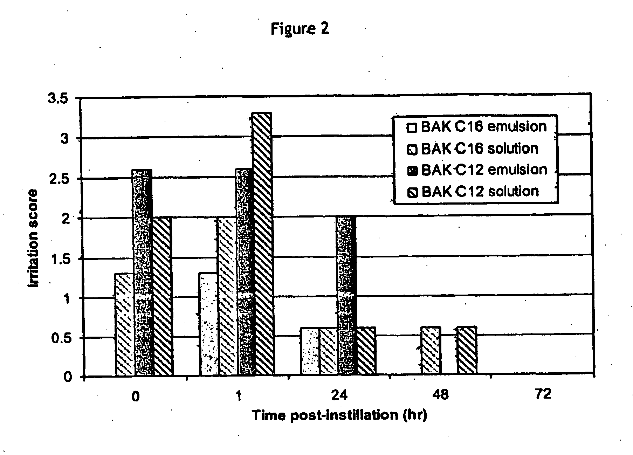

The graph (figure 2) shows that the incorporation of BAK C16 within an emulsion results in an unexpected decrease of its toxicity.

### 6. Emulsions containing therapeutically active compound

Emulsions loaded with a therapeutically active compound (0.05% w/w Cyclosporin A) and containing different amounts and chain lengths of BAK were prepared as described previously.

| | w/w | Emulsion | Zeta potential (mV) |
|---|---|---|---|
| BAK C16 | 0.002% | EM067 | +23.0 |
| | 0.0025% | EM063 | +23.2 |
| | 0.003% | EM070 | +26.7 |
| | 0.005% | EM064 | +29.2 |
| 40% BAK | 0.005% | EM065 | +19.6 |
| C12 | 0.01% | EM066 | +27.9 |
| 30% BAK | | | |
| C14 | | | |
| 30% BAK | | | |
| C16 | | | |

## Claims

1. Oil-in-water emulsion comprising a quaternary ammonium halide composition, said composition comprising at least one quaternary ammonium halide in which the nitrogen atom is substituted by one alkyl group having at least 12 carbon atoms, **characterized in that** said composition includes:
a) at least 20% in weight by weight of the total composition of quaternary ammonium halides in which the nitrogen atom is substituted by one alkyl group having at least 14 carbon atoms and
b) more than 5 %, preferably more than 7 % in weight by weight of the total composition of quaternary ammonium halides in which the nitrogen atom is substituted by one alkyl group having at least 16 carbon atoms.

2. Oil-in-water emulsion according to claim 1, wherein said composition includes
a) at least 20% in weight by weight of the total composition of quaternary ammonium halides in which the nitrogen atom is substituted by one alkyl group having at least 14 carbon atoms and
b) at least 10%, preferably at least 15%, more preferably at least 20 % in weight by weight of the total composition of quaternary ammonium halides in which the nitrogen atom is substituted by one alkyl group having at least 16 carbon atoms.

3. Oil-in-water emulsion according to anyone of claims **1** or **2,** wherein said quaternary ammonium halides are benzyl dimethyl ammonium chlorides or bromides, wherein the nitrogen atom is further substituted by one alkyl group having at least 12 carbon atoms.

4. Oil-in-water emulsion according to anyone of claims **1** to **3,** comprising C14- and C16-alkyl benzyl dimethyl ammonium chlorides.

5. Oil-in-water emulsion according to anyone of claims **1** to **4,** wherein said quaternary ammonium halide is a trimethyl ammonium chloride or bromide, wherein the nitrogen atom is further substituted by an alkyl group having at least 12 carbon atoms.

6. Oil-in-water emulsion According to anyone of claims **1** to **5,** wherein the amount of ammonium halides in which the nitrogen atom substituted by at least one alkyl group having 14 or 16 carbon atoms represents at least 50% w/w in dry weight of the weight of all ammonium halides present in the composition.

7. Oil-in-water emulsion according to anyone of claims **1** to **6,** wherein the dry weight ratio of C12-alkyl ammonium halides to the sum of C14-alkyl ammonium halides and C16-alkyl ammonium halides, is less than 1.5, preferably less than 1.35, more preferably less than 1.20.

8. Oil-in-water emulsion according to anyone of claims **1** to **7,** said emulsion comprising 0.0005 to 0.1 % of quaternary ammonium halides.

9. Oil-in-water emulsion according to anyone of claim 1 to 8, said emulsion further comprising hydroxypropyl guar, polyethylene glycol 400 or a mixture of both.

10. Oil-in-water emulsion according to any of claims 1 to 9, further comprising an oil phase comprising MCT or castor oil or mineral oil, surfactants preferably chosen among at least one of tyloxapol, poloxamer, tocopherol, polyethylene glycol succinate and polysorbate, and optionally antioxidants and/or isotonicity agents preferably chosen among at least one of glycerol and mannitol.

11. Oil-in-water emulsion according to anyone of claim **1** to **10,** said emulsion having a positive zeta potential.

12. Oil-in-water emulsion according to anyone of claims **1** to **11,** said emulsion having a droplet size of 100 to 500 nm.

13. Oil-in-water emulsion according to anyone of claim **1** to **12,** said emulsion being preserved.

14. Oil-in-water emulsion according to anyone of claim **1** to **12,** said emulsion being unpreserved.

15. Oil-in-water emulsion according to anyone of claim **1** to **3,** said emulsion comprising C16-alkyl quaternary ammonium halide as only source of quaternary ammonium halide.

16. Oil-in-water emulsion according to anyone of claim **1** to **14,** further comprising an active principle.

17. Medicament comprising of an oil-in-water emulsion according to anyone of claims **1** to **16.**

18. Use of an oil-in-water emulsion according to anyone of claims **1** to **16** for the manufacture of a medicament or an ophthalmic composition for the treatment of an eye condition or eye disease.

19. Cosmetic composition comprising an oil-in-water emulsion according to anyone of claims **1** to **16.**

20. A non-therapeutical process for caring for, removing makeup from and/or cleansing the skin, the lips and/or the eyes, and/or for hair care, comprising applying an oil-in-water emulsion according to anyone of claims **1** to **16** onto the skin, the lips, the eyes, and/or the hair.

## Patentansprüche

1. Öl-in-Wasser-Emulsion, umfassend eine quaternäre Ammoniumhalogenid-Zusammensetzung, wobei die Zusammensetzung mindestens ein quaternäres Ammoniumhalogenid umfasst, in dem das Stickstoffatom durch eine Alkylgruppe substituiert ist, die mindestens 12 Kohlenstoffatome aufweist, **dadurch gekennzeichnet, dass** die Zusammensetzung Folgendes umfasst:
a) mindestens 20 Massenprozent der Gesamtzusammensetzung an quaternären Ammoniumhalogeniden, in denen das Stickstoffatom durch eine Alkylgruppe substituiert ist, die mindestens 14 Kohlenstoffatome aufweist, und
b) mehr als 5, vorzugsweise mehr als 7 Massenprozent der Gesamtzusammensetzung an quaternären Ammoniumhalogeniden, in denen das Stickstoffatom durch eine Alkylgruppe substituiert ist, die mindestens 16 Kohlenstoffatome aufweist.

2. Öl-in-Wasser-Emulsion nach Anspruch **1**, wobei die Zusammensetzung Folgendes umfasst:
a) mindestens 20 Massenprozent der Gesamtzusammensetzung an quaternären Ammoniumhalogeniden, in denen das Stickstoffatom durch eine Alkylgruppe substituiert ist, die mindestens 14 Kohlenstoffatome aufweist, und
b) mindestens 10, vorzugsweise mindestens 15, insbesondere mindestens 20 Massenprozent der Gesamtzusammensetzung an quaternären Ammoniumhalogeniden, in denen das Stickstoffatom durch eine Alkylgruppe substituiert ist, die mindestens 16 Kohlenstoffatome aufweist.

3. Öl-in-Wasser-Emulsion nach einem der Ansprüche **1** oder **2**, wobei die quaternären Ammoniumhalogenide Benzyldimethylammoniumchloriden oder -bromiden sind, wobei das Stickstoffatom weiter durch eine Alkylgruppe substituiert ist, die mindestens 12 Kohlenstoffatome aufweist.

4. Öl-in-Wasser-Emulsion nach einem der Ansprüche **1** bis **3**, umfassend C14- und C16-Alkylbenzyldimethylammoniumchloriden.

5. Öl-in-Wasser-Emulsion nach einem der Ansprüche **1** bis **4**, wobei das besagte quaternäre Ammoniumhalogenid ein Trimethylammoniumchlorid oder -bromid ist, wobei das Stickstoffatom weiter durch eine Alkylgruppe substituiert ist, die mindestens 12 Kohlenstoffatome aufweist.

6. Öl-in-Wasser-Emulsion nach einem der Ansprüche **1** bis **5**, wobei die Menge von Ammoniumhalogeniden, in denen das Stickstoffatom, substituiert durch mindestens eine Alkylgruppe mit 14 oder 16 Kohlenstoffatomen, mindestens 50 % w/w an Trockengewicht des Gewichts aller Ammoniumhalogenide darstellt, die in der Zusammensetzung vorhanden sind.

7. Öl-in-Wasser-Emulsion nach einem der Ansprüche **1** bis **6**, wobei das Trockengewichtsverhältnis von C12-Alkylammoniumhalogeniden zur Summe aus C14-Alkylammoniumhalogeniden und C16-Alkylammoniumhalogeniden weniger als 1,5, vorzugsweise weniger als 1,35 und vorzugsweise weniger als 1,20 ist.

8. Öl-in-Wasser-Emulsion nach einem der Ansprüche **1** bis **7**, wobei die Emulsion 0,0005 bis 0,1 % an quaternären Ammoniumhalogeniden umfasst.

9. Öl-in-Wasser-Emulsion nach einem der Ansprüche **1** bis **8**, wobei die Emulsion weiter Hydroxypropylguar, Polyethylenglykol 400 oder eine Mischung aus beiden umfasst.

10. Öl-in-Wasser-Emulsion nach einem der Ansprüche **1** bis **9**, weiter umfassend eine Ölphase, umfassend MCT oder Rizinusöl oder Mineralöl, oberflächenaktive Stoffe, vorzugsweise ausgewählt aus mindestens einem von Tyloxapol, Poloxamer, Tocopherol, Polyethylenglycolsuccinat und Polysorbat, und optional Antioxidationsmitteln und/oder sotonizitätsmitteln, vorzugsweise ausgewählt aus mindestens einem von Glycerol und Mannitol.

11. Öl-in-Wasser-Emulsion nach einem der Ansprüche **1** bis **10,** wobei die Emulsion ein positives Zeta-Potential aufweist.

12. Öl-in-Wasser-Emulsion nach einem der Ansprüche **1** bis **11,** wobei die Emulsion eine Tröpfchengröße von 100 bis 500 nm aufweist.

13. Öl-in-Wasser-Emulsion nach einem der Ansprüche **1** bis **12,** wobei die Emulsion konserviert ist.

14. Öl-in-Wasser-Emulsion nach einem der Ansprüche **1** bis **12,** wobei die Emulsion nicht konserviert ist.

15. Öl-in-Wasser-Emulsion nach einem der Ansprüche **1** bis **3,** wobei die Emulsion C-16-Alkyl quaternäre Ammoniumhalogenid als einzige Quelle von quaternärem Ammoniumhalogenid umfasst.

16. Öl-in-Wasser-Emulsion nach einem der Ansprüche **1** bis **14,** weiter umfassend einen Wirkstoff.

17. Arzneimittel, umfassend eine Öl-in-Wasser-Emulsion nach einem der Ansprüche **1** bis **16.**

18. Verwendung einer Öl-in-Wasser-Emulsion nach einem der Ansprüche **1** bis **16** zur Herstellung eines Arzneimittels oder einer ophthalmischen Zusammensetzung für die Behandlung eines Augenzustandes oder einer Augenkrankheit.

19. Kosmetische Zusammensetzung, umfassend eine Öl-in-Wasser-Emulsion nach einem der Ansprüche **1** bis **16.**

20. Nicht therapeutisches Verfahren zur Pflege, zur Entfernung von Make-up von und/oder zur Reinigung der Haut, der Lippen und/oder der Augen und/oder für die Haarpflege, umfassend die Anwendung einer Öl-in-Wasser-Emulsion nach einem der Ansprüche **1** bis **16** auf die Haut, die Lippen, die Augen und/oder die Haare.

## Revendications

1. Émulsion huile-dans-eau comprenant une composition d'halogénure d'ammonium quaternaire, ladite composition comprenant au moins un halogénure d'ammonium quaternaire dans lequel l'atome d'azote est substitué par un group alkyle ayant au moins 12 atomes de carbone, **caractérisé en ce que** ladite composition inclut :
a) au moins 20% en poids par rapport au poids de la composition totale d'halogénures d'ammonium quaternaire dans lesquels l'atome d'azote est substitué par un group alkyle ayant au moins 14 atomes de carbone, et
b) plus de 5%, de préférence plus de 7% en poids par rapport au poids de la composition totale d'halogénures d'ammonium quaternaire dans lesquels l'atome d'azote est substitué par un groupe alkyle ayant au moins 16 atomes de carbone.

2. Émulsion huile-dans-eau selon la revendication **1,** dans laquelle ladite composition inclut :
a) au moins 20% en poids par rapport au poids de la composition totale d'halogénures d'ammonium quaternaire dans lesquels l'atome d'azote est substitué par un groupe alkyle ayant au moins 14 atomes de carbone, et
b) au moins 10%, de préférence au moins 15%, plus préférentiellement au moins 20% en poids par rapport au poids de la composition totale d'halogénures d'ammonium quaternaire dans lesquels l'atome d'azote est substitué par un groupe alkyle ayant au moins 16 atomes de carbone.

3. Émulsion huile-dans-eau selon l'une quelconque des revendications **1** ou **2,** dans laquelle lesdits halogénures d'ammonium quaternaire sont des chlorures ou bromures de benzyle diméthyle ammonium, dans lesquels l'atome d'azote est en outre substitué par un groupe alkyle ayant au moins 12 atomes de carbone.

4. Émulsion huile-dans-eau selon l'une quelconque des revendications **1** à **3,** comprenant des chlorures de C14- et C16-alkyle benzyle diméthyle ammonium.

5. Émulsion huile-dans-eau selon l'une quelconque des revendications **1** à **4,** dans laquelle ledit halogénure d'ammonium quaternaire est un chlorure ou bromure de triméthyle ammonium, dans lequel l'atome d'azote est en outre substitué par un groupe alkyle ayant au moins 12 atomes de carbone.

6. Émulsion huile-dans-eau selon l'une quelconque des revendications **1** à **5,** dans laquelle la quantité d'halogénures d'ammonium dans lesquels l'atome d'azote est substitué par au moins un groupe alkyle ayant 14 ou 16 atomes de carbone représente au moins 50 % p/p en poids sec par rapport au poids de tous les halogénures d'ammonium présents dans la composition.

7. Émulsion huile-dans-eau selon l'une quelconque des revendications **1** à **6,** dans laquelle le ratio en poids sec des halogénures de C12-alkyle ammonium sur la somme des halogénures de C14-alkyle ammonium et des halogénures de C16-alkyle ammonium, est inférieur à 1,5, de préférence inférieur à 1,35, plus préférentiellement inférieur à 1,20.

8. Émulsion huile-dans-eau selon l'une quelconque des revendications **1** à **7,** ladite émulsion comprenant 0,0005 à 0,1% d'halogénures d'ammonium quaternaire.

9. Émulsion huile-dans-eau selon l'une quelconque des revendications **1** à **8,** ladite émulsion comprenant en outre du guar hydroxypropylé, du polyéthylène glycol 400 ou un mélange des deux.

10. Émulsion huile-dans-eau selon l'une quelconque des revendications **1** à **9,** comprenant en outre une phase huileuse comprenant du MCT ou de l'huile de ricin ou de l'huile minérale, des surfactants de préférence choisis parmi au moins l'un de tyloxapol, poloxamère, tocophérol, succinate de polyéthylène glycol et polysorbate, et optionnellement des antioxydants et/ou des agents isotoniques choisis de préférence parmi au moins un de glycérol et mannitol.

11. Émulsion huile-dans-eau selon l'une quelconque des revendications **1** à **10,** ladite émulsion ayant un potentiel zéta positif.

12. Émulsion huile-dans-eau selon l'une quelconque des revendications **1** à **11,** ladite émulsion ayant une taille de gouttelettes de 100 à 500 nm.

13. Émulsion huile-dans-eau selon l'une quelconque des revendications **1** à **12,** ladite émulsion étant conservée.

14. Émulsion huile-dans-eau selon l'une quelconque des revendications **1** à **12,** ladite émulsion n'étant pas conservée.

15. Émulsion huile-dans-eau selon l'une quelconque des revendications **1** à **3,** ladite émulsion comprenant un halogénure de C16-alkyle ammonium quaternaire comme seule source d'halogénure d'ammonium quaternaire.

16. Émulsion huile-dans-eau selon l'une quelconque des revendications **1** à **14,** comprenant en outre un principe actif.

17. Médicament comprenant une émulsion huile-dans-eau selon l'une quelconque des revendications **1** à **16.**

18. Utilisation d'une émulsion huile-dans-eau selon l'une quelconque des revendications **1** à **16** pour la fabrication d'un médicament ou d'une composition ophtalmique pour le traitement d'une condition de l'oeil ou d'une maladie de l'oeil.

19. Composition cosmétique comprenant une émulsion huile-dans-eau selon l'une quelconque des revendications **1** à **16.**

20. Un procédé non thérapeutique pour prendre soin de, pour le démaquillage de et/ou le nettoyage de la peau, des lèvres et/ou des yeux, et/ou pour le soin des cheveux, comprenant l'application d'une émulsion huile-dans-eau selon l'une quelconque des revendications **1** à **16** sur la peau, les lèvres, les yeux, et/ou les cheveux.
